# EUROPÄISCHE PATENTSCHRIFT

(11) **EP 4 012 405 B1**
(45) Veröffentlichungstag und Bekanntmachung des Hinweises auf die Patenterteilung: **15.07.2026**
(21) Anmeldenummer: 21211193.4
(22) Anmeldetag: 30.11.2021
(51) Int. Cl.: G01N 33/00

(54) **GASMESSGERÄT**
GAS MEASURING DEVICE
APPAREIL DE MESURE DE GAZ

(30) Priorität: 09.12.2020 DE 102020132771
(43) Veröffentlichungstag der Anmeldung: 15.06.2022
(73) Patentinhaber: Dräger Safety AG & Co. KGaA, 23560 Lübeck (DE)
(72) Erfinder: Tschuncky, Peter, 23558 Lübeck (DE); Gimbel, Doreen, 23558 Lübeck (DE); Gnoerrlich, Tim, 23558 Lübeck (DE); Veelken, Henrik, 23558 Lübeck (DE)
(74) Vertreter: Weber-Lehn, Christian

(56) Entgegenhaltungen:
- EP-A1- 1 281 957
- WO-A1-2019/086200
- DE-A1- 102009 052 957
- DE-A1- 19 708 052
- DE-U1- 202006 020 536
- GB-A- 2 356 708
- US-A- 5 667 558
- US-B1- 6 635 160

## Beschreibung

Die Erfindung betrifft ein Gasmessgerät, einen Tester für ein Gasmessgerät und ein Testverfahren zum Testen eines Gasmessgeräts.

Gasmessgeräte, insbesondere Gaswarneinrichtungen mit einem Gassensor müssen in regelmäßigen Abständen Funktionstests unterzogen werden, um deren korrekte Funktionalität sicherzustellen.

Beim Betrieb eines Gasmessgeräts kann es beispielsweise durch Verstopfung einer Öffnung, durch die das Gasmessgerät mit einem Umgebungsmedium in Verbindung steht, oder durch mechanische bzw. elektrische Störungen zu einer eingeschränkten Funktionalität des Gasmessgeräts kommen.

Um eine eingeschränkte Funktionalität eines Gasmessgeräts zu erkennen und das Gasmessgerät reparieren oder austauschen zu können, sind Verfahren bekannt, bei denen ein Gassensor des Gasmessgeräts durch einen Gaskanal mit einem Testgas beaufschlagt und eine entsprechende Sensorantwort des Gassensors ausgewertet wird. Aufgrund der Verwendung eines einzelnen Gaskanals ist ein Einfluss von Störvariablen bei sämtlichen Sensorantworten nicht exakt zu bestimmen, wodurch es ggf. zu fehlerhaften Messungen kommen kann.

So beschreibt die US 2008/0282765 A1 einen Gassensor mit einem einzelnen Gaskanal, mindestens einem Gassensor, einem Gasgenerator und einer Pumpe. Mittels der Pumpe wird von dem Gasgenerator erzeugtes Testgas zu dem Gassensor geleitet, um diesen zu testen.

Die US 5 667 558 A beschreibt einen Gaswäscher mit einem Auslassgassensor und einer Pumpe zum Zuführen von Chemikalien in eine Waschsuspension zum Waschen eines Gases.

Die US 4 742 708 A beschreibt ein elektrochemisches Gaserfassungssystem mit einem elektrochemischen Sensor und einem Gehäuse mit einem Reservoir für einen Elektrolyten. Ferner umfasst das Gaserfassungssystem ein Kalibrierungssystem mit einer Kalibrierungsgasquelle zum Kalibrieren des Gaserfassungssystems.

Die DE 10 2009 052 957 A1 beschreibt eine Vorrichtung mit einem Gassensor in einem Sensorgehäuse, welches eine gaspermeable Membran für die zu analysierende Gasprobe und eine Messelektrode aufweist, und einem Prüfgasgenerator, welcher ein Generatorgehäuse besitzt, das im Bereich der gaspermeablen Membran befestigt ist, eine Gaszutrittsöffnung für die Gasprobe hat und zur gaspermeablen Membran hin gerichtete Auslassöffnungen für Prüfgas aufweist.

Die GB 2 356 708 A beschreibt ein Sensorsystem, das Folgendes umfasst: ein Gehäuse, das mindestens einen Gassensor enthält, wobei das Gehäuse mindestens eine Öffnung aufweist, so dass Gas zu dem Sensor gelangen kann; eine Prüfgaseinrichtung zum Bereitstellen eines Prüfgases, das verwendet wird, um zu überprüfen, ob der Sensor funktioniert; und eine Steuereinrichtung zum Analysieren der Reaktion des Sensors auf mindestens das Prüfgas.

Die DE 20 2006 020 536 U1 beschreibt einen Gasgenerator mit einer Elektrolysezelle mit einem Gehäuse, welches durch eine gasdurchlässige Membran für den Austritt des Prüf- oder Kalibriergases verschlossen ist; einer Kathode aus einem Edelmetall, einem Gemisch aus Edelmetallen oder aus einem Kohlenstoff enthaltenden Material, welche in direktem Kontakt mit einem Elektrolyten steht; einer Anode aus einem Edelmetall, einem Gemisch aus Edelmetallen oder aus einem Kohlenstoff enthaltenden Material, welche in direktem Kontakt mit einem Elektrolyten steht, wobei im Elektrolyten ein Alkalimetallsalz, ein Erdalkalimetallsalz, ein Ammoniumsalz entweder der Essigsäure oder einer Dicarbonsäure enthalten ist; einer auch als Stromquelle dienenden Steuereinheit, welche mit den Elektroden verbunden ist.

Die DE 197 08 052 A1 beschreibt eine Vorrichtung zum automatischen Kalibrieren von Gassensoren durch Beaufschlagung des Sensors mit einem Kalibriergas, wobei sich der Sensor in einer Meßkammer befindet, die mit einer Öffnung zum Entweichen des Kalibriergases und einer Gaszuleitung versehen ist, wobei in der Nähe des Sensors ein Kalibriergasbehälter angeordnet ist, der mit einem von einem Prozeßor gesteuerten Thermoventil versehen ist, wobei der Kalibriergasbehälter aus einem Sammelbehälter besteht, der mit einer Mikrobohrung versehen ist und an dem ein Zwischenbehälter mit einem mehrfach betätigbarem elektrisch beheizten Zinn-Schmelz-Verschluß angeordnet ist.

Die WO 2019 / 086 200 A1 beschreibt eine Testvorrichtung mit einen Gasgenerator zum Erzeugen von Testgases und mit einer Steuereinheit zum Ansteuern des Gasgenerators sowie zum Ermitteln einer Sensorantwort des Gassensors.

Es ist eine Aufgabe der vorliegenden Erfindung, einen verbesserten Tester und ein verbessertes Gasmessgerät bereitzustellen. Es ist insbesondere die Aufgabe der vorliegenden Erfindung, eine Möglichkeit zum Testen der aktuellen Funktionalität eines Gasmessgeräts bereitzustellen.

Voranstehende Aufgabe wird gelöst durch die Merkmale der unabhängigen Ansprüche. Weitere Merkmale und Details der Erfindung ergeben sich aus den Unteransprüchen, der Beschreibung und den Zeichnungen. Dabei gelten Merkmale und Details, die im Zusammenhang mit dem Gasmessgerät oder dem Tester beschrieben sind, selbstverständlich auch im Zusammenhang mit dem erfindungsgemäßen Testverfahren und jeweils umgekehrt, sodass bezüglich der Offenbarung zu den einzelnen Erfindungsaspekten stets wechselseitig Bezug genommen wird beziehungsweise Bezug genommen werden kann.

Es wird somit ein Gasmessgerät vorgestellt.

Das Gasmessgerät umfasst mindestens einen einen Analyten umsetzenden chemischen Gassensor mit einem umgebenden Sensorraum, mindestens einen Öffnungsbereich, der eine gasdurchlässige Verbindung zwischen dem Sensorraum und einer Umgebung des Gasmessgerätes bildet, und mindestens einen erfindungsgemäßen Tester.

Der Tester umfasst eine Testeinheit und eine Schnittstelle zum reversiblen mechanischen und/oder kommunikativen Verbinden der Testeinheit mit einem Gassensor. Die Testeinheit umfasst eine Gaskanalanordnung mit einem ersten Gaskanal und einem zweiten Gaskanal, und mindestens einen Gasgenerator, der dazu eingerichtet ist, mindestens ein Testgas zu einem ersten Zeitabschnitt eines Tests der Testeinheit und zu einem zweiten Zeitabschnitt des Tests über die Gaskanalanordnung in den umgebenden Sensorraum des Gassensors zu leiten. Dabei ist die Testeinheit eingerichtet, das mindestens eine Testgas zu dem ersten Zeitabschnitt über den ersten Gaskanal zu dem Sensorraum zu leiten und das mindestens eine Testgas zu dem zweiten Zeitabschnitt über den zweiten Gaskanal zu dem Sensorraum zu leiten. Dabei unterscheidet sich der erste Gaskanal von dem zweiten Gaskanal in seiner Kanalstruktur, so dass ein Testgastransport während des ersten Zeitabschnitts eine andere Transportcharakteristik aufweist als während des zweiten Zeitabschnitts.

Unter dem Begriff "Test" ist im Kontext der vorgestellten Erfindung eine Prozedur zum Validieren oder Überprüfen einer Funktionalität zu verstehen. Entsprechend wird bei einem Test überprüft, ob eine vorgegebene Funktion korrekt durchgeführt und/oder bereitgestellt wird.

Unter einem Testgas ist im Kontext der vorgestellten Erfindung ein Gas mit einer bekannten, vorgegebenen Zusammensetzung und Konzentration von in dem Testgas enthaltenen Stoffen zu verstehen.

Unter einer Kanalstruktur eines Gaskanals ist eine strukturelle Charakteristik dieses Gaskanals, wie beispielsweise Form, Größe, Querschnittsfläche, Oberflächencharakteristik und/oder Länge des Gaskanals zu verstehen.

Unter einem Gasgenerator ist im Kontext der vorgestellten Erfindung eine Vorrichtung bestehend aus mindestens einer Generatorelektrode und einer Gegenelektrode zu verstehen, wobei bei Verwendung mehrerer Gasgeneratoren eine gemeinsame Gegenelektrode vorgesehen sein kann. Der Gasgenerator kann dafür auch unter Verwendung einer geeigneten Tablette, wie etwa einer Silbersulfidtablette, ein Testgas generieren.

Der erfindungsgemäße Öffnungsbereich kann beispielsweise durch einen Filter gegenüber größeren Partikeln abgeschirmt sein, jedoch einen Übertritt von Umgebungsmedium, insbesondere Umgebungsluft, ermöglichen. Typischerweise ist der Gassensor außerhalb des Tests über die Testeinheit zum Detektieren einer Gaskomponente in dem über den Öffnungsbereich eintretenden Umgebungsmedium, insbesondere Umgebungsluft, ausgebildet.

Der Sensorraum ist ein Raum, der den Gassensor umgibt und dabei für das Umgebungsmedium mit dem auszuwertenden Analyten über den mindestens einen Öffnungsbereich zugänglich ist. Der Sensorraum wird dabei vorzugsweise durch ein Sensorgehäuse des Gasmessgerätes räumlich begrenzt.

Unter einem Referenzwert ist im Kontext der vorgestellten Erfindung eine Anzahl vorgegebener Werte zu verstehen, die mathematisch zu einem Vergleichswert in Beziehung gesetzt werden kann. Ein Referenzwert kann einen einzelnen oder eine Vielzahl Werte, insbesondere einen Kurvenverlauf, umfassen. Ein Referenzwert kann positive und/oder negative Werte umfassen und insbesondere als Band eines Wertebereichs angegeben sein. Ein Referenzwert kann eine mathematische Verarbeitung und/oder Verdichtung eines oder mehrerer Werte sein.

Unter einer Recheneinheit ist im Kontext der vorgestellten Erfindung jede programmierbare Vorrichtung zu verstehen. Insbesondere kann eine Recheneinheit ein Schaltkreis, wie beispielsweise ein ASIC, mindestens ein Prozessor oder ein verteiltes System sein. Insbesondere ist eine Recheneinheit ein Computer.

Das vorgestellte Gasmessgerät besteht aus mindestens einem chemischen Gassensor, der ein den Gassensor anströmendes Gas chemisch umsetzt, um eine Sensorantwort zu generieren, und mindestens einer Testeinheit. Der Gassensor kann ein Pellistor, also ein Wärmeströmungssensor, der ein Gas verbrennt und eine entstehende Wärmeströmung misst oder ein elektrochemischer Sensor sein, der ein Gas reduziert oder oxidiert und elektrische Eigenschaften des reduzierten oder oxidierten Gases misst.

Die erfindungsgemäß vorgesehene Testeinheit umfasst vorzugsweise eine Struktur, wie etwa eine Basis, die aus einem vorzugsweise gasundurchlässigen Material, wie beispielsweise Kunststoff, bestehen kann. Die Basis kann insbesondere eine runde, vorzugsweise eine kreisförmige Form aufweisen und mit der Gaskanalanordnung verbunden sein. Die Gaskanalanordnung kann beispielsweise kreuzförmig innerhalb einer kreisförmigen Basis angeordnet sein.

Die Testeinheit ist insbesondere dazu konfiguriert, eine Funktionalität des Gasmessgeräts zu testen. Dazu kann die Testeinheit eine Funktionalität, also eine jeweilige Funktion des Gasmessgeräts, insbesondere des Gassensors testen, indem der Gassensor durch einen Gasgenerator mit einem Testgas begast wird.

Zum Begasen des Gassensors umfasst die Testeinheit die Gaskanalanordnung, mit der Anzahl von Gaskanälen, also mindestens den ersten Gaskanal und den zweiten Gaskanal, mit einer jeweiligen Kanalstruktur. Über diese Anzahl von Gaskanälen erfolgt der Testgastransport zum ersten Zeitabschnitt des Tests der Testeinheit und zum zweiten Zeitabschnitt des Tests der Testeinheit. Die beiden Zeitabschnitte haben vorzugsweise keinen oder einen messtechnisch vernachlässigbaren überschneidenden Zeitbereich. Alternativ kann ein Überscheiden der beiden Zeitbereiche für den erfindungsgemäßen Test durch die Testeinheit vorgesehen sein.

Erfindungsgemäß ist neben dem ersten Zeitabschnitt und dem zweiten Zeitabschnitt auch das Vorsehen mindestens eines weiteren Zeitabschnitts für einen Test durch die Testeinheit in einer erfindungsgemäßen Ausgestaltung des Gasmessgerätes möglich.

Durch die unterschiedliche Transportcharakteristik der jeweiligen Gaskanäle verändert sich ein Konzentrations-Zeit-Profil eines zu detektierenden Gases am Ort der Detektion im Gassensor und damit die Sensorantwort des Gassensors beim Begasen mit Testgas während der zwei unterschiedlichen Zeitabschnitte. Dies hat zur Folge, dass durch eine Begasung des Gassensors mit Testgas während der jeweiligen Zeitabschnitte unterschiedliche Gaswolken mit unterschiedlichen Konzentrations-Zeit-Profilen entstehen, die aufgrund dieser Unterschiede auch unterschiedlich durch Umgebungsparameter wie beispielsweise Wind beeinflusst werden können. Insbesondere kann durch eine Begasung des Gassensors mit Testgas während des ersten Zeitabschnitts eine Funktionalität des Gassensors und durch eine Begasung des Gassensors mit Testgas während des zweiten Zeitabschnitts eine Funktionalität der Öffnung des Gasmessgeräts getestet werden.

Weiterhin können Sensorwerte, die von dem Gassensor bei einer Begasung des Gassensors mit Testgas während des ersten Zeitabschnitts ermittelt wurden und Sensorwerte, die bei einer Begasung des Gassensors mit Testgas während des zweiten Zeitabschnitts ermittelt wurden, miteinander in eine mathematische Beziehung gesetzt werden, um auf Eigenschaften des Gasmessgeräts oder einer Umgebung des Gasmessgeräts zu schließen.

Insbesondere können sich die beiden Transportcharakteristiken während der beiden Zeitabschnitte durch unterschiedliche Wechselwirkungen oder unterschiedliche Einflüsse einer Umgebung auf in das durch die Gaskanalanordnung strömende Gas unterscheiden. Vorzugsweise sind dazu der erste Gaskanal und mindestens ein zweiter Gaskanal der Gaskanalanordnung unterschiedlich konstruiert.

Die Gaskanalanordnung kann grundsätzlich neben dem ersten und zweiten Gaskanal auch mindestens einen weiteren Gaskanal aufweisen.

Es kann vorgesehen sein, dass sich der erste und zweite Gaskanal dadurch unterscheiden, dass eine erste Gaskanalöffnung des ersten Gaskanals näher an dem Gassensor liegt als eine zweite Gaskanalöffnung des zweiten Gaskanals.

Alternativ oder ergänzend kann vorgesehen sein, dass die zweite Gaskanalöffnung näher an dem mindestens einen Öffnungsbereich liegt als die erste Gaskanalöffnung.

Mittels einer unterschiedlichen Kanalstrukturen der jeweiligen Gaskanäle des vorgestellten Gasmessgeräts können Testgase mit unterschiedlichen Strömungseigenschaften und dadurch mit einer unterschiedlichen Transportcharakteristik zum Begasen des Gassensors erzeugt werden. Dabei kann beispielsweise ein erster Gaskanal, der eine Gaskanalgeometrie aufweist, die dicht vor dem Gassensor endet, zum Testen des Gassensors an sich verwendet werden, während ein Gaskanal, der eine Gaskanalgeometrie aufweist, die relativ zu dem ersten Gaskanal weiter beabstandet von dem Gassensor endet, dazu verwendet werden kann, ein Strömungsverhalten des Öffnungsbereiches und/oder eines Sensoreingangs des Gasmessgeräts zu testen.

Bei einem geringen Abstand zwischen einem Ende eines Gaskanals und einem Gassensor nehmen Umgebungseinflüsse, wie beispielsweise durch Umgebungswind bedingte Nebenströmungen, minimalen Einfluss auf entsprechende Sensorwerte. Entsprechend eignet sich eine derartige Gaskanalgeometrie besonders vorteilhaft zum Testen der Funktionsfähigkeit und der Größe der Empfindlichkeit des Gassensors, da eine geringe Störvarianz und/oder ein geringer Einfluss von Störvariablen bei einem entsprechenden Test zu erwarten ist.

Da bei einem großen Abstand zwischen einem Ende eines Gaskanals und einem Gassensor Umgebungseinflüsse, wie beispielsweise durch Umgebungswind bedingte Nebenströmungen, einen starken Einfluss auf entsprechende Sensorwerte haben und vorzugsweise ein Öffnungsbereich und/oder ein Sensoreingang des Gasmessgeräts näher an dem Ende des Gaskanals liegt als der Gassensor, eignet sich eine derartige Gaskanalgeometrie besonders vorteilhaft zum Testen eines Öffnungsbereiches und/oder eines Sensoreingangs des Gasmessgeräts. Insbesondere eignet sich eine derartige Gaskanalgeometrie besonders vorteilhaft zum Testen eines Öffnungsbereiches und/oder eines Sensoreingangs des Gasmessgeräts, da ein Verstopfungsgrad einer Öffnung die Transportcharakteristik des entsprechenden Testgases zwischen Ende des Gaskanals und Gassensor besonders stark beeinflusst.

Es kann weiterhin vorgesehen sein, dass der erste Gaskanal und der mindestens eine weitere Gaskanal sich durch eine Rauheit ihrer Innenoberfläche in ihrer Kanalstruktur voneinander unterscheiden.

Mittels einer unterschiedlichen Rauheit von Innenbereichen der jeweiligen Gaskanäle des vorgestellten Gasmessgeräts können Testgase mit unterschiedlicher Transportcharakteristik, insbesondere unterschiedlichen Strömungseigenschaften, zum Begasen des Gassensors erzeugt werden. Dabei kann beispielsweise ein erster Gaskanal, dessen Innenoberfläche eine niedrige Rauheit aufweist, also besonders glatt ist, zum Testen des Gassensors an sich verwendet werden, während ein Gaskanal, der eine Innenoberfläche mit einer relativ zu dem ersten Gaskanal höheren Rauheit aufweist, dazu verwendet werden kann, ein Strömungsverhalten des Öffnungsbereiches des Gasmessgeräts zu testen.

Da bei einer niedrigen Rauheit einer Innenoberfläche eines Gaskanals ein durch den Gaskanal strömendes Testgas sehr schnell strömt, nehmen Umgebungseinflüsse, wie beispielsweise durch Umgebungswind bedingte Nebenströmungen, einen geringeren Einfluss auf die kompakte Gaswolke am Austrittsloch des Gaskanals als dies bei einer gebremsten, diffusen Gasabgabe im Austrittsbereich eines rauen Kanals der Fall wäre. Entsprechend eignet sich ein derartiger Gaskanal besonders vorteilhaft zum Testen der Funktionsfähigkeit und der Größe der Empfindlichkeit des Gassensors, da eine geringe Störvarianz und somit ein geringer Einfluss von Störvariablen bei einem entsprechenden Test zu erwarten ist.

Da bei einer großen Rauheit einer Innenoberfläche eines Gaskanals ein durch den Gaskanal strömendes Testgas beispielsweise aufgrund von Adsorbtions und Desorptionsvorgängen an den Wänden des Gaskanals langsamer strömt, entsteht am Ausgang eine zeitlich verzögert auftretende Gaswolke, die zeitlich gestreckt und mit niederer Konzentration am Ort der Detektion auftritt und damit andersartig durch Umgebungseinflüsse, wie beispielsweise Wind, beeinflusst wird. Entsprechend eignet sich ein derartiger Gaskanal besonders vorteilhaft zum Testen einer Öffnung des Gasmessgeräts, da deren Verstopfungsgrad mit einem Einfluss von durch Umgebungswind bedingten Nebenströmungen mathematisch verbunden und dadurch korreliert ist.

Es kann weiterhin vorgesehen sein, dass der mindestens eine Gaskanal eine Temperierungseinheit umfasst, die dazu konfiguriert ist, eine Temperatur im Inneren des Gaskanals derart zu ändern, dass sich das Testgas zum ersten Zeitabschnitt von dem Testgas zum zweiten Zeitabschnitt in seiner Gastemperatur unterscheidet. Das Testgas zum ersten Zeitabschnitt kann dabei ein von dem Testgas zum zweiten Zeitabschnitt verschiedenes Testgas sein.

Mittels einer unterschiedlichen Temperierung des Testgases des vorgestellten Gasmessgeräts können Testgase mit unterschiedlicher Transportcharakteristik zum Begasen des Gassensors erzeugt werden. Dabei kann beispielsweise ein erster Gaskanal, mit einer Temperiereinheit zum Erwärmen des ersten Gaskanals zum Testen des Gassensors verwendet werden, während ein anderer Gaskanal der Gaskanalanordnung, der keine Temperiereinheit oder eine Temperiereinheit zum Kühlen des Gaskanals aufweist, dazu verwendet werden kann, ein Strömungsverhalten der Öffnung des Gasmessgeräts zu testen.

Da bei einer hohen Temperatur eines Gaskanals ein durch den Gaskanal strömendes Testgas schnell strömt, tritt das Testgas als kompakte Gaswolke aus dem Gaskanal aus und Umgebungsatmosphäreneinflüsse, wie beispielsweise Wind, haben einen geringeren Einfluss auf die kompakte Gaswolke am Austrittsloch des Gaskanals als dies bei einer niedrigen Temperatur des Gaskanals der Fall wäre. Entsprechend eignet sich ein erwärmter und/oder erwärmbarer Gaskanal besonders vorteilhaft zum Testen des Gassensors, da eine geringe Störvarianz also ein geringer Einfluss von Störvariablen bei einem entsprechenden Test zu erwarten ist.

Da bei einer niedrigen Temperatur eines Gaskanals ein durch den Gaskanal strömendes Testgas langsam strömt, entsteht am Ausgang eine zeitlich verzögert auftretende Gaswolke, die zeitlich gestreckt und mit niederer Konzentration am Ort der Detektion auftritt und damit andersartig durch Umgebungseinflüsse, wie beispielsweise Wind, beeinflusst und/oder beinflussbar wird. Entsprechend eignet sich ein gekühlter und/oder kühlbarer Gaskanal besonders vorteilhaft zum Testen einer Öffnung des Gasmessgeräts gegenüber der Umgebungsatmosphäre, da deren Verstopfungsgrad mit einem Einfluss von durch Umgebungswind bedingten Nebenströmungen mathematisch verbunden und dadurch korreliert ist.

Es kann weiterhin vorgesehen sein, dass der erste Gaskanal und der mindestens eine weitere Gaskanal insbesondere über jeweilige Kammern der Gaskanäle miteinander in fluider Verbindung stehen und der erste Gaskanal nicht in direktem fluidem Kontakt zu einer Umgebung der Testeinheit steht und der mindestens eine weitere Gaskanal in direktem fluidem Kontakt zu der Umgebung der Testeinheit steht.

Um einen Einfluss von umgebungsbedingten Einflüssen auf ein Testgas zum Testen eines Gassensors zu minimieren, eignet sich besonders vorteilhaft ein Gaskanal mit einer Kammer, die nicht in direktem fluidem Kontakt mit einer Umgebung steht.

Um einen Einfluss von umgebungsbedingten Einflüssen auf ein Testgas zum Testen eines Gassensors zu maximieren, eignet sich besonders vorteilhaft ein Gaskanal mit einer Kammer, die in direktem fluidem Kontakt mit einer Umgebung steht.

In einer bevorzugten Ausführungsform ist der mindestens eine Gasgenerator ausgebildet, während des ersten Zeitabschnitts ein anderes Testgas durch den entsprechenden Gaskanal zu leiten als während des zweiten Zeitabschnitts. Die zwei verschieden Testgase weisen besonders bevorzugt zwei unterschiedliche Diffusionskoeffizienten auf. Hierdurch werden unabhängig von der Struktur der Gaskanalanordnung verschiedenen Transportcharakteristiken ermöglichst, insbesondere unterschiedliche Konzentrations-Zeit-Profile.

**In** einer Ausführungsform liegt zwischen dem ersten Zeitabschnitt und dem zweiten Zeitabschnitt ein Zeitintervall, in dem kein Testgas von dem Gasgenerator bereitgestellt wird, wobei dieses Zeitintervall mindestens 1 Minute, insbesondere mindestens 30 Minuten, besonders bevorzugt mindestens 2 Stunden beträgt. In dieser Ausführungsform erfolgt eine klare zeitliche Trennung zwischen einer ersten Phase des Tests während des ersten Zeitabschnitts und einer zweiten Phase des Tests während des zweiten Zeitabschnitts. Dadurch können chemische Prozesse, die während des ersten Zeitabschnitts erfolgen besonders zuverlässig abgewartet werden, ehe während des zweiten Zeitabschnitts eine erneute Phase des Tests des Gasmessgerätes erfolgt.

Die Testeinheit des vorgestellten Gasmessgeräts kann mindestens eine Recheneinheit umfassen. Die mindestens eine Recheneinheit ist vorzugsweise ausgebildet, ein Signal des Gassensors zu empfangen und daraus einen Messwert zu ermitteln, wobei die Recheneinheit weiterhin ausgebildet ist, während des ersten Zeitabschnitts und/oder während des zweiten Zeitabschnitts eine Testinformation zu bestimmen und auszugeben, wobei die Testinformation indiziert, ob das Gasmessgerät fehlerhaft ist, und wobei das Bestimmen der Testinformation auf einem Vergleich zwischen dem Messwert und einem vorbestimmten Gassensorschwellenwert oder zwischen einem bestimmten Messwertverlaufsparameter und einem vorbestimmten Verlaufsparameter basiert. Dabei kann die Testinformation beispielsweise indizieren, ob der mindestens eine Öffnungsbereich verstopft ist und/oder ob der Gassensor funktioniert. **In** den folgenden Ausführungsformen sind verschiedenen Beispiele für die mögliche Funktionsweise einer solchen Recheneinheit angegeben. Die Verwendung eines Vergleichs zwischen vorbestimmten Messwerten oder einem vorbestimmten Messwertverlauf ermöglicht eine besonders einfache und zuverlässige Bestimmung der Testinformation durch die Recheneinheit.

In einem Beispiel der vorhergehenden Ausführungsform ist die Recheneinheit ausgebildet, für einen der beiden Zeitabschnitte den Vergleich zwischen dem Messwert und einem vorbestimmten Gassensorschwellenwert auszuführen und für den anderen der beiden Zeitabschnitte den Vergleich zwischen dem Messwertverlaufsparameter und dem vorbestimmten Verlaufsparameter auszuführen. Hierdurch werden während der jeweiligen Zeitabschnitte unterschiedliche Bestimmungsvorschriften ausgeführt, die im Ergebnis zu der Testinformation führen.

Alternativ oder ergänzend kann eine Auswertung über eine Mehrzahl von zurückliegenden Messwerten aus ersten und/oder zweiten Zeitabschnitten durch die Recheneinheit erfolgen. Eine derartige Berücksichtigung der Messwert-Historie kann zu einer besonders zuverlässigen Auswertung führen.

Die Recheneinheit kann alternativ oder ergänzend mindestens ein Steuerungsmodul zum Steuern von Komponenten des Gasmessgeräts, wie beispielsweise den Gasgenerator umfassen. Alternativ oder zusätzlich kann die mindestens eine Recheneinheit ein Analysemodul zum Auswerten jeweiliger Messwerte des Gassensors des vorgestellten Gasmessgeräts umfassen. Insbesondere dient die mindestens eine Recheneinheit zum Betrieb des vorgestellten Gasmessgeräts. Dazu kann die mindestens eine Recheneinheit mit dem Gassensor und/oder jeweiligen Gasgeneratoren kommunikativ verbunden sein, um mit dem Gassensor und/oder den jeweiligen Gasgeneratoren Kontrollbefehle auszutauschen. Die mindestens eine Recheneinheit kann einen oder mehrere Prozessoren umfassen, die zum Betreiben des vorgestellten Gasmessgeräts konfiguriert sind. Die mindestens eine Recheneinheit kann in einem Computer, wie beispielsweise einem Server, insbesondere einer Cloud, verortet sein und mit dem vorgestellten Gasmessgerät über eine Kommunikationsschnittstelle in kommunikativer Verbindung stehen. Alternativ kann die mindestens eine Recheneinheit zumindest teilweise in dem Gasmessgerät selbst verortet sein.

Es kann weiterhin vorgesehen sein, dass die erfindungsgemäß vorgesehene Recheneinheit dazu konfiguriert ist, für den Fall, dass eine Differenz oder ein Quotient von während des ersten Zeitabschnitts durch den Gassensor zu ermittelnden Messwerten oder daraus berechenbaren Zwischenergebnissen und mindestens einem vorgegebenen Referenzwert größer ist als ein vorgegebener Gassensorschwellenwert, den Gassensor oder das Gasmessgerät als fehlerhaft zu markieren, und für den Fall, dass eine Differenz oder ein Quotient einer die Signalgröße beschreibenden Variablen wie der Abklingzeit, der Peakhöhe, des Integrals oder daraus berechenbaren Zwischenergebnissen von während des zweiten Zeitabschnitts durch den Gassensor ermittelten Messwerten zu einem vorgegebenen Referenzwert größer ist als ein vorgegebener Verstopfungsschwellenwert, den Öffnungsbereich, durch die der Gaskanal mit der Umgebung in direkter Verbindung steht, als verstopft zu markieren.

Mittels eines mathematischen Abgleichs von während einer Begasung mit Testgas durch den erfindungsgemäß vorgesehenen Gaskanal ermittelten Messwerten und/oder einem durch deren mathematische Weiterverarbeitung erhaltenen Wert und einem vorgegebenen Referenzwert kann eine Funktionalität des Gassensors getestet werden.

Da der erfindungsgemäß vorgesehene Gassensor ein chemischer Gassensor ist, setzt dieser bei korrekter also fehlerfreier Funktionalität das Testgas chemisch um, sodass eine Konzentration des Testgases über die Zeit absinkt und von dem Gassensor ermittelte Messwerte sich entsprechend ändern.

Sollte der Abgleich zwischen jeweiligen bei einer Begasung während des ersten Zeitabschnitts ermittelten Messwerten des Gassensors und einem Referenzwert keine oder eine nur geringe Änderung der Messwerte ergeben, indem beispielsweise eine Differenz oder ein Quotient zwischen den Messwerten und/oder einem durch deren mathematische Weiterverarbeitung erhaltenen Wert und dem Referenzwert über einem vorgegebenen Gassensorschwellenwert liegt, kann auf eine fehlerhafte Funktionalität des Gassensors oder des Gasmessgeräts geschlossen werden. Entsprechend ist die erfindungsgemäß vorgesehene Recheneinheit dazu konfiguriert, in einem solchen Falle den Gassensor oder das Gasmessgerät als fehlerhaft zu markieren und beispielsweise eine Fehlermeldung in einem Speicher des Gassensors, des Gasmessgerätes und/oder in einem Fehlerspeicher und/oder in einem Speicher der Recheneinheit zu hinterlegen. Dabei kann der Referenzwert beispielsweise ein Sensorwert zu einer Halbwertszeit einer Abbaukurve eines Referenzsensors sein oder sich auf die Entwicklung der Kenngröße des jeweiligen Sensors selbst beziehen.

Mittels eines mathematischen Abgleichs einer während einer Begasung mit Testgas während des zweiten Zeitabschnitts ermittelten Kenngröße wie beispielsweise einer Abklingzeit, einer Peakhöhe, eines Integrals oder weiterer Kenngrößen und einem vorgegebenen Referenzwert kann eine Funktionalität der Öffnung des Gasmessgeräts getestet werden. Da durch die Öffnung Nebenströmungen, wie beispielsweise Umgebungsluft, in das Gasmessgerät strömen und/oder Testgas aus dem Gasmessgerät strömt, wird bei einer nicht verstopften Öffnung eine Konzentration eines Testgases über die Zeit minimiert, sodass eine Konzentration des Testgases über die Zeit absinkt und von dem Gassensor ermittelte Messwerte sich entsprechend schnell ändern, eignet sich eine Kenngröße, die die Höhe und den Umfang des Signals beschreibt,, besonders vorteilhaft zum Testen einer Funktionalität der Öffnung. Eine solche Kenngröße wäre beispielsweise die Peakhöhe in Kombination mit der Abklingzeit oder die umgesetzte Menge an Gas am Sensor, die über das Integral dargestellt werden kann.

Zur Überprüfung der ordnungsgemäßen Empfindlichkeit eines Gassensors kann der Gassensor mittels des erfindungsgemäßen Gasgenerators mit Testgas beaufschlagt werden. Dabei erfolgt die Beaufschlagung zunächst derart, dass ein beispielsweise erster Gaskanal, der von jeweiligen Umgebungsbedingungen möglichst unabhängig ist und/oder nicht direkt mit einer Umgebung in Kontakt steht, benutzt wird.

Zur Beurteilung, ob ein ungehinderter Gaszutritts zu einem zu testenden Gassensor erfolgen kann und/oder zur Feststellung von eventuellen Blockaden oder Verstopfungen, wird Testgas mittels des erfindungsgemäßen Gasgenerators beispielsweise durch einen zweiten Gaskanal zu dem Gassensor geleitet, der eine besonders starke Interaktion des Testgases mit der Umgebungsatmosphäre fördert und vorzugsweise in direktem Kontakt oder lediglich durch einen Filter getrennt mit einer Umgebung ist.

Infolge der Gaszuleitung durch den erfindungsgemäß vorgesehenen Gasgenerator reagiert der Gassensor sowohl bei einer Begasung durch den ersten Gaskanal als auch bei einer Begasung durch den zweiten Gaskanal mit einem Sensorsignal, das proportional zur Konzentration des zugeleiteten Testgases ist. Ein dabei entstehender zeitlicher Verlauf von durch den Gassensor ermittelten Messwerten wird durch eine Analyseeinheit für eine Begasung durch den ersten Gaskanal und eine Begasung durch den zweiten Gaskanal ausgewertet.

Zum einen ist es bei der Auswertung der Messwerte des Gaskanals möglich, einen Abgleich zwischen einer generellen Kurvenform eines Konzentrations-Zeit-Profils des Gassensors und einem vorgegebenen beispielsweise ein einem Speicher der Analyseeinheit hinterlegten Referenzwert in Form einer Fitfunktion durchzuführen. Durch einen solchen Abgleich ist es möglich, eine qualitative Bewertung der Kurvenform der von dem Gassensor ermittelten Messwerte zu erhalten. Ein derartiger Abgleich mit einer Fitfunktion bildet eine Kinetik eines Messsignals des Gassensors gegen die Zeit auf Basis einer normierten Kurve oder einer Kurve beispielsweise in Konzentrationseinheiten ab. Hierzu können insbesondere geeignete Fitparameter einer mathematischen Funktion herangezogen werden.

Zur quantitativen Bewertung der Messwerte des Gaskanals können mathematische Kenngrößen der Messwerte wie Maximum, Minimum, Halbwertsbreite, Halbwertszeit eines abfallenden Astes der Messwerte, eine Signalhöhe der Messwerte bspw. in Konzentrationseinheiten zu bestimmten Zeitpunkten auf dem abfallenden Ast, Flächenintegrale mit unterschiedlichen Grenzen, Standardabweichungen über unterschiedliche Wertebereiche, Mediane sowie jede weitere technische geeignete mathematische Kenngröße verwendet werden.

Insbesondere ist vorzugsweise der durch die Recheneinheit ausgeführte Vergleich zum Bestimmen der Testinformation von einer auf dem ermittelten Messwertverlauf basierenden Bestimmung einer Abklingzeit, einer mathematischen Ableitung, eines statistischen Mittels, eines Maximalwerts und/oder eines Integrals sowie einer Betrachtung früherer Messwerte abhängig.

Weiterhin ist es möglich, durch Extrapolation aus Teilabschnitten der Messwerte des Gassensors charakteristische Größen abzuleiten, die ein Verhalten des Gassensors hinreichend beschreiben. Beispielsweise kann in einer Auftragung von Messwerten in Konzentrationseinheiten gegen die Zeit aus einer linearen Extrapolation über einen selektierten Wertebereich zwischen zwei Zeitpunkten vor Erreichen eines Maximalwertes eine Information über ein Ansprechverhalten, Ansprechzeiten und Totzeiten des Sensors ermittelt werden. Entsprechend kann nach Erreichen des Maximalwertes eine Information über ein Abklingverhalten des Sensors und eine Unverstopftheit eines Gaszutritts des Gassensors ermittelt werden. Weitere geeignete Auftragungen der Messwerte des Gassensors können herangezogen werden, um die Funktionalität des Gassensors zu bewerten. Beispielsweise können die Messwerte des Gassensors gegen eine Gasdosis, also einem Integral über einen zeitlichen Verlauf der Messwerte, aufgetragen und ausgewertet werden um die Testinformation zu bestimmen.

Insbesondere können ermittelte Istwerte, also Messwerte des Gassensors und Referenzwerte durch geeignete mathematische Verfahren miteinander verglichen werden. Die Referenzwerte können beispielsweise als Toleranzbänder vorgegeben werden, die Bereiche für ordnungsgemäß arbeitende Gassensoren und Gasgeneratoren beschreiben. Bei Abweichungen oder Übertretungen der Messwerte des Gassensors aus diesen Toleranzbändern, kann auf verschiedene Zustände oder Fehlfunktionen des Gassensors geschlossen werden. Zum Beispiel kann der Gassensor zu unempfindlich, zu empfindlich, zu langsam und zu schnell auf das Testgas reagieren, das Testgas kann zu schnell oder zu langsam abtransportiert und/oder weiterverarbeitet werden. Insbesondere kann ein Kurvenverlauf der Messwerte gegenüber einer vorgegebenen Referenzkurve zeitlich oder in seinen Konzentrationswerten verschoben sein. Jede Abweichung von einem vorgegebenen Referenzwert wird entsprechend einer in einer Auswerteeinheit hinterlegten Auswertelogik bewertet und gegebenenfalls nach erfolgtem Wiederholtest einem Endnutzer ausgegeben. Insbesondere kann anhand einer Auswertung der Messwerte des Gassensors eine Handlungsempfehlung für einen Nutzer ausgegeben werden, insbesondere im Rahmen der Testinformation ausgegeben werden.

Sollte der Abgleich zwischen der ermittelten Abklingzeit und dem Referenzwert eine langsame Änderung der Messwerte ergeben, indem beispielsweise eine Differenz zwischen der Abklingzeit und dem Referenzwert oberhalb eines vorgegebenen Gassensorschwellenwerts liegt, kann auf eine fehlerhafte Funktionalität der Öffnung und/oder eine Verstopfung geschlossen und das Gasmessgerät entsprechend als fehlerhaft markiert werden. Entsprechend ist die erfindungsgemäß vorgesehene Recheneinheit dazu konfiguriert, in einem solchen Fall die Öffnung, insbesondere den Öffnungsbereich, oder das Gasmessgerät als fehlerhaft zu markieren und beispielsweise eine Fehlermeldung in einem Speicher des Gasmessgeräts und/oder in einem Fehlerspeicher und/oder in einem Speicher der Recheneinheit zu hinterlegen. Dabei kann der Referenzwert beispielsweise ein ermittelter Sensorwert zu einer Abklingzeit, insbesondere einer Halbwertszeit einer Abbaukurve eines Referenzsensors in einer Referenzkammer mit einer vollständig durchgängigen Öffnung sein.

Es kann weiterhin vorgesehen sein, dass die Recheneinheit dazu konfiguriert ist, für den Fall, dass Messwerte, die bei einer Begasung des Gassensors mit Testgas durch einen Gaskanal, dessen Innenoberfläche eine Rauheit aufweist, die geringer ist als die Rauheit einer Innenoberfläche eines jeweilig anderen Gaskanals, nach einem vorgegebenen Zeitpunkt größer oder kleiner sind als ein vorgegebener Referenzwert, das Gasmessgerät durch eine entsprechende Testinformation als fehlerhaft zu markieren und für den Fall, dass Messwerte, die bei einer Begasung des Gassensors mit Testgas durch den jeweilig anderen Gaskanal nach einem vorgegebenen Zeitpunkt größer oder kleiner sind als ein vorgegebener Referenzwert, eine Öffnung, durch die der jeweilig andere Gaskanal mit einer Umgebung des Gasmessgeräts in direktem fluiden Kontakt steht, über die entsprechende Testinformation als verstopft zu markieren.

Da bei einem fehlerhaften Gassensor eine Umsetzung eines Testgases gestört ist und die Konzentration des Testgases sich, dadurch bedingt, nur minimal ändert, kann bei Messwerten, die zu einem Zeitpunkt ermittelt werden, zu dem sich eine Umsetzung des Testgases bei einem Referenzsensor auf die Konzentration des Testgases auswirkt auf eine Funktionalität des Gassensors geschlossen werden und dadurch die entsprechende Testinformation ausgegeben werden. Dazu können die Messwerte des Gassensors zu diesem Zeitpunkt ausgewertet werden, sodass für den Fall, dass diese Messwerte größer sind als ein vorgegebener Referenzwert oder die Messwerte sich von einem Maximum der Konzentration des Testgases nur um einen Betrag unterscheiden, der unterhalb oder je nach Vorzeichen eines entsprechenden Messsignals, oberhalb eines vorgegebenen Schwellenwerts liegt, davon ausgegangen werden kann, dass der Gassensor und entsprechend das Gasmessgerät fehlerhaft ist.

Da bei einem Test des Gassensors Störvariablen zu minimieren sind, eignet sich eine Begasung durch einen für einen besonders schnellen Gastransport geeigneten Gaskanal, insbesondere durch einen Gaskanal mit einer besonders glatten Innenoberfläche, in besonders vorteilhafter Weise zum Testen des Gassensors.

Da bei einem Test der Öffnung des Gasmessgeräts eine Wechselwirkung zwischen einem Testgas und einer Umgebung betrachtet wird, eignet sich eine Begasung des Gassensors durch einen Gaskanal, der dazu führt, dass das Testgas besonders lange mit einer Umgebung wechselwirken kann. Dazu kann sich insbesondere ein Gaskanal mit einer besonders rauen Innenoberfläche eignen. Durch einen Gaskanal mit einer rauen Innenoberfläche wird das Testgas verlangsamt und strömt entsprechend langsam und, dadurch bedingt, mit einer langen Zeit zum Wechselwirken mit einem Umgebungsmedium, zu dem Gassensor.

Es kann weiterhin vorgesehen sein, dass die Recheneinheit dazu konfiguriert ist, für den Fall, dass Messwerte, die bei einer Begasung des Gassensors mit Testgas durch einen Gaskanal, dessen Innentemperatur höher ist als eine Innentemperatur eines jeweilig anderen Gaskanals, nach einem vorgegebenen Zeitpunkt größer oder je nach Vorzeichen eines entsprechenden Messsignals kleiner, sind als ein vorgegebener Referenzwert, den Gassensor und/oder das Gasmessgerät über die Testinformation als fehlerhaft zu markieren und für den Fall, dass Messwerte, die bei einer Begasung des Gassensors mit Testgas durch den jeweilig anderen Gaskanal nach einem vorgegebenen Zeitpunkt größer oder entsprechend kleiner sind als ein vorgegebener Referenzwert, eine Öffnung, durch die der jeweilig andere Gaskanal mit einer Umgebung des Gasmessgeräts in direktem fluiden Kontakt steht, über die Testinformation als verstopft zu markieren.

Da bei einem Test des Gassensors Störvariablen zu minimieren sind, eignet sich eine Begasung durch einen für einen besonders schnellen Gastransport geeigneten Gaskanal, insbesondere durch einen besonders warmen Gaskanal, in besonders vorteilhafter Weise zum Testen des Gassensors.

Da bei einem Test der Öffnung des Gasmessgeräts eine Wechselwirkung zwischen einem Testgas und einer Umgebung betrachtet wird, eignet sich eine Begasung des Gassensors durch einen für einen besonders langsamen Gastransport geeigneten Gaskanal, insbesondere durch einen besonders kalten Gaskanal, in besonders vorteilhafter Weise zum Testen der Öffnung.

Es kann weiterhin vorgesehen sein, dass die Recheneinheit dazu konfiguriert ist, für den Fall, dass eine Differenz von während mindestens eines ersten Zustands durch den Gassensor zu ermittelnden Messwerten und mindestens einem vorgegebenen Referenzwert größer oder kleiner ist als ein vorgegebener Gassensorschwellenwert, den Gassensor anhand der Differenz zu rekalibrieren.

Um einen als fehlerhaft markierten Gassensor zu reparieren oder den Gassensor kontinuierlich an eine aktuelle Situation anzupassen, kann der Gassensor rekalibriert werden. Dazu kann vorgesehen sein, dass beispielsweise eine Vergleichstabelle oder eine Zuordnungslogik die jeweilige von dem Gassensor ermittelte Messwerte jeweiligen auszugebenden Werten einer Ausgabeskala zuordnet, in Abhängigkeit einer Abweichung zwischen während des ersten Zustands durch den Gassensor ermittelten Messwerten und einem vorgegebenen Referenzwert angepasst wird. Dazu kann beispielsweise eine Warnschwelle, ab der ein Warnton auszugeben ist, in Abhängigkeit der Abweichung angehoben oder absenkt werden. In diesem Sinne ist ein Warnton auch eine Testinformation. Allgemein ist eine Testinformation eine Information, die ein Ergebnis des Tests während mindestens einem der beiden erfindungsgemäßen Zeitabschnitte indiziert.

Insbesondere kann vorgesehen sein, dass ein Trend von durch den Gassensor ermittelten Messwerten verwendet wird, um den Gassensor zu rekalibrieren. Dazu kann beispielsweise ein Mittelwert oder jeder weitere technisch geeignete Kennwert einer Vielzahl von zeitlich versetzt ermittelten Messwerten gebildet werden.

Es kann weiterhin vorgesehen sein, dass der erste Gaskanal derart gestaltet ist, dass das Testgas schneller durch den ersten Gaskanal strömt als durch den zweiten Gaskanal, und dass die Recheneinheit dazu konfiguriert ist, eine Differenz einer ersten Zeit zwischen einem Start einer Begasung mit Testgas durch den ersten Gaskanal und einem Zeitpunkt einer Erhöhung von Messwerten des Gassensors sowie einer weiteren Zeit zwischen einem Start einer Begasung mit Testgas durch den mindestens einen weiteren Gaskanal und einem Zeitpunkt einer Erhöhung von Messwerten des Gassensors zu ermitteln und anhand der Differenz auf eine Diffusionszeit des Testgases zum Messgeräts zu schließen und diese Diffusionszeit beim Durchführen eines Tests zu berücksichtigen.

Durch Ermitteln einer solchen Diffusionszeit, die sich vorzugsweise in Abhängigkeit einer Umgebungstemperatur ändern kann, können von der Recheneinheit des Gasmessgeräts zu berechnende Größen, wie zum Beispiel eine Abklingzeit oder jede weitere zu berechnende oder zu ermittelnde Größe, wie beispielsweise eine Temperatur, korrigiert werden.

Es kann weiterhin vorgesehen sein, dass die Recheneinheit dazu konfiguriert ist, anhand eines Abgleichs von während des ersten Zeitabschnitts durch den Gassensor zu ermittelnden Messwerten und während des zweiten Zeitabschnitts durch den Gassensor zu ermittelnden Messwerten auf eine Windgeschwindigkeit außerhalb des Öffnungsbereichs zu schließen.

Durch einen Abgleich von Messwertens des Gassensors, die bei einer Begasung mit Testgas ermittelt wurden, das durch Verwendung eines Gases schnell leitenden Gaskanales erhalten wurde und Messwerten des Gassensors, die bei einer Begasung mit Testgas mittels eines langsam Gas leitenden Gaskanales erhalten wurde, kann auf Eigenschaften einer mit dem Testgas wechselwirkenden Störvariable, insbesondere auf eine Geschwindigkeit einer durch die Öffnung einströmenden Nebenströmung, geschlossen werden. Entsprechend kann anhand des Abgleichs auf eine Windgeschwindigkeit außerhalb des Gasmessgeräts also außerhalb des Öffnungsbereichs geschlossen werden. Dabei gilt, je stärker auftretende Abweichungen von Referenzwerten sind, desto schneller ist die Windgeschwindigkeit.

Es kann weiterhin vorgesehen sein, dass die Testeinheit einen ersten Gasgenerator und zumindest einen weiteren Gasgenerator umfasst, und die Recheneinheit dazu konfiguriert ist, für den Fall, dass der Gassensor bei einer Begasung durch den ersten Gasgenerator als fehlerhaft zu markieren ist, den Gassensor durch den mindestens einen weiteren Gasgenerator mit Testgas zu begasen und
für den Fall, dass der Gassensor bei der Begasung mit Testgas durch den weiteren Gasgenerator nicht als fehlerhaft zu markieren ist, den Gassensor als fehlerfrei und den ersten Gasgenerator als fehlerhaft zu markieren und die entsprechende Testinformation bereitzustellen.

Mittels mehrerer Gasgeneratoren kann beispielsweise ein Validierungsgasgenerator, der vorzugsweise lediglich dann eingesetzt wird, wenn ein Gassensor als fehlerhaft zu markieren ist, dazu verwendet werden, den Gasgenerator, der einem Test zugrunde liegt, der zu dem Ergebnis geführt hat, dass der Gassensor oder die Öffnung als fehlerhaft zu markieren ist, überprüft werden. Entsprechend kann durch den Validierungsgasgenerator eine falsch fehlerhafte Markierung eines Gassensors oder Gasmessgeräts vermieden werden.

Es kann weiterhin vorgesehen sein, dass der erste Gaskanal und der zweite Gaskanal parallel nebeneinander angeordnet oder zu mindestens einem Labyrinth verbunden sind.

Durch ein Labyrinth an Gaskanälen können Umgebungseinflüsse auf jeweilige mittels des Gassensors zu ermittelnde Messwerte minimiert werden.

Der erfindungsgemäße Tester ist vorzugsweise reversibel mit einem Gasmessgerät und/oder einem Gassensor verbindbar, sodass der Tester zum Testen einer Vielzahl von Gasmessgeräten eingesetzt werden kann. Entsprechend wird hinsichtlich des Testers auf die im Rahmen des vorgestellten Gasmessgeräts beschriebenen Vorteile Bezug genommen.

In einem weiteren Aspekt betrifft die vorgestellte Erfindung ein Testverfahren zum Testen eines Gasmessgeräts. Das Testverfahren umfasst die folgenden Schritte:
a) Bereitstellen eines Gasmessgeräts nach einer der vorhergehenden Ausführungsformen;
b) Ansteuern des mindestens einen Gasgenerators derart, dass dieser mindestens ein Testgas zu einem ersten Zeitabschnitt eines Tests und zu einem zweiten Zeitabschnitt des Tests über die Gaskanalanordnung in den umgebenden Sensorraum des Gassensors leitet;
c) Ermitteln von durch den Gassensor während des ersten Zeitabschnitts und/oder während des zweiten Zeitabschnitts ermittelten Messwerten;
d) Bestimmen und Ausgeben einer Testinformation während des ersten Zeitabschnitts und/oder während des zweiten Zeitabschnitts, wobei die Testinformation indiziert, ob das Gasmessgerät fehlerhaft ist, und wobei das Bestimmen der Testinformation auf einem Vergleich zwischen dem Messwert oder einem daraus berechneten Kennwert und einem vorbestimmten Gassensorschwellenwert oder zwischen einem bestimmten gemäß einer vorbestimmten Vorschrift berechneten Messwertverlaufsparameter und einem vorbestimmten Verlaufsparameter basiert.

Das vorgestellte Testverfahren dient insbesondere zum Betrieb des vorgestellten Gasmessgeräts sowie des vorgestellten Testers, sodass hinsichtlich der Vorteile des vorgestellten Verfahrens auf die beschriebenen Vorteile des Gasmessgeräts und/oder des Testers Bezug genommen wird.

Weitere, die Erfindung verbessernde Maßnahmen ergeben sich aus der nachfolgenden Beschreibung zu einigen Ausführungsbeispielen der Erfindung, welche in den Figuren dargestellt sind. Sämtliche aus den Ansprüchen, der Beschreibung oder der Zeichnung hervorgehende Merkmale und/oder Vorteile, einschließlich konstruktiver Einzelheiten und räumlicher Anordnungen können sowohl für sich als auch in den verschiedenen Kombinationen erfindungswesentlich sein. Es zeigen jeweils schematisch:
- Figur 1: eine geschnittene Seitenansicht einer möglichen Ausgestaltung des erfindungsgemäßen Gasmessgeräts,
- Figur 2: einen Vergleich von Messdaten, die unter Verwendung des Gasmessgeräts aus Figur 1 bei einer Begasung durch einen ersten Gaskanal ermittelt wurden und Messdaten, die bei einer Begasung durch einen zweiten Gaskanal ermittelt wurden,
- Figur 3: einen Vergleich von Messdaten, die unter Verwendung des Gasmessgeräts aus Figur 1 zum Ermitteln des Einflusses einer Umgebungswindgeschwindigkeit verwendet werden können,
- Figur 4: eine weitere mögliche Ausgestaltung des vorgestellten Gasmessgeräts, und
- Figur 5: einen Ablauf einer möglichen Ausgestaltung des erfindungsgemäßen Verfahrens.

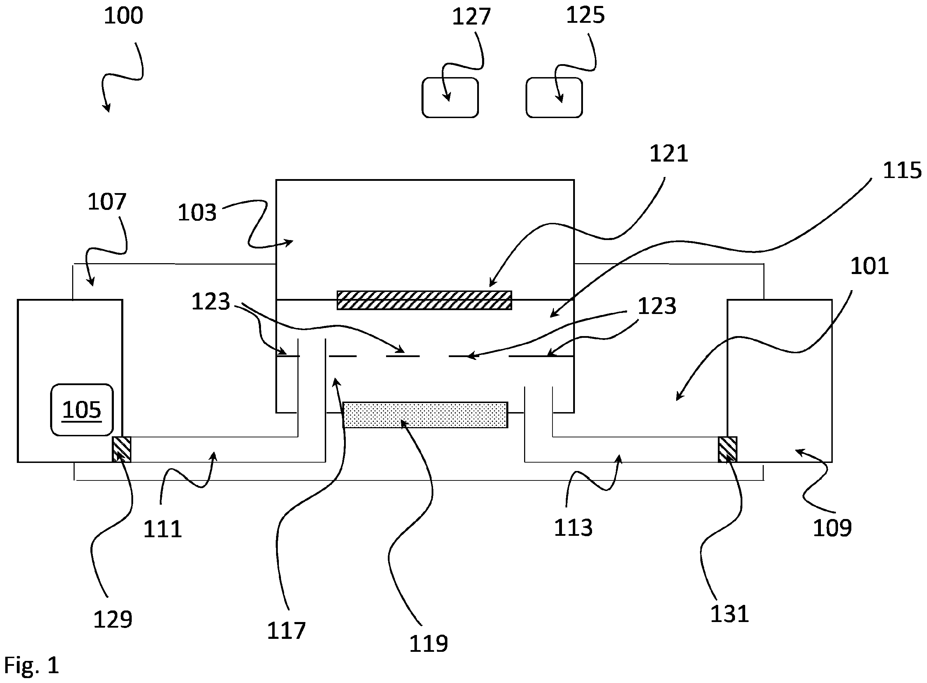

In Fig. 1 ist ein Gasmessgerät 100 in einer geschnittenen Seitenansicht dargestellt. Das Gasmessgerät umfasst eine Testeinheit 101 und einen Gassensor 103.

Die Testeinheit 101 umfasst einen insbesondere chemischen Gasgenerator 105 zum Umsetzen eines Analyten eines Testgases zum Testen des Gasmessgeräts 100.

Der Gasgenerator 105 besteht aus einer Generatorelektrode in einer ersten Generatorkammer 107 und einer Gegenelektrode. Ferner ist eine weitere Generatorenkammer 109 vorgesehen. Insbesondere kann vorgesehen sein, dass die Gegenelektrode gemeinsam von mehreren Gasgeneratoren verwendet wird.

Die erste Generatorkammer 107 steht über eine erste Gasaustrittsöffnung 129 mit einem ersten Gaskanal 111 in Kontakt, sodass von dem Gasgenerator 105 erzeugtes Testgas durch die erste Gasaustrittsöffnung 129 in den ersten Gaskanal 111 strömt. Um einen Austritt von ggf. in der ersten Generatorkammer 107 gespeichertem Elektrolyt in den ersten Gaskanal 111 zu verhindern, umfasst die erste Gasaustrittsöffnung 129 eine gaspermeable Membran, die für das Testgas durchlässig und für den Elektrolyten dicht ist.

Die weitere Generatorkammer 109 steht über eine weitere Gasaustrittsöffnung 131 mit einem zweiten Gaskanal 113 in Kontakt, sodass von dem optional weiteren Gasgenerator und/oder einer optionalen weiteren Generatorenelektrode erzeugtes Testgas durch die weitere Gasaustrittsöffnung 131 in den zweiten Gaskanal 113 strömt, um einen Austritt von ggf. in der weiteren Generatorkammer 109 gespeichertem Elektrolyt in den zweiten Gaskanal 113 zu verhindern, umfasst die weitere Gasaustrittsöffnung 131 eine selektiv gaspermeable Membran, die für das Testgas durchlässig und für den Elektrolyten dicht ist.

Die Testeinheit 101 ist hier fest mit dem Gassensor 103 verbunden. Zusätzlich ist die Testeinheit 101 derart ausgestaltet, dass sie eine Schnittstelle zum kommunikativen und/oder mechanischen Verbinden mit dem Gassensor 103 aufweist.

Die Testeinheit 101 kann als kreisrunde Basis ausgestaltet sein, an der der erste Gaskanal 111 und der zweite Gaskanal 113 angeordnet sind oder in die der erste Gaskanal 111 und der zweite Gaskanal 113 integriert sind.

Der erste Gaskanal 111 umfasst eine erste Kammer 115.

Der zweite Gaskanal 113 umfasst eine weitere Kammer 117 mit einem Öffnungsbereich 119, durch den die Kammer 117 über einen Filter mit einer Umgebung in Kontakt steht, sodass Umgebungsmedium, wie bspw. Luft in die Kammer 117 einströmen bzw. Testgas aus der Kammer 117 ausströmen kann. Die erste Kammer 115 und die weitere Kammer 117 stehen strömungstechnisch im Austausch, sodass Gase von der weiteren Kammer 117 in die erste Kammer 115 strömen können. Die Strömungsbewegung zwischen der ersten Kammer 115 und der weiteren Kammer 117 wird durch Strömungsleitvorrichtungen 123 geführt und/oder begrenzt. Die erste Kammer 115 und die weitere Kammer 117 bilden in dem dargestellten Ausführungsbeispiel einen den Gassensor 103 umgebenden Sensorraum.

Der erste Gaskanal 111 hat eine Kanalstruktur, die sich von der Kanalstruktur des zweiten Gaskanals 113 unterscheidet. Vorliegend ist der erste Gaskanal 111 länger als der zweite Gaskanal 113, sodass ein Abstand zwischen einem Ende des ersten Gaskanals 111 und einem Einlassbereich 121 des Gassensors 103, kürzer ist als ein Abstand zwischen einem Ende des zweiten Gaskanals 113 und dem Einlassbereich 121 des Gassensors 103.

Weiterhin umfasst die Testeinheit 101 eine erste Recheneinheit 125, die als Steuerungsmodul dazu konfiguriert ist, den Gasgenerator 105 und den Gassensor 103 zu steuern. Dazu kann die erste Recheneinheit 125 mit dem Gasgenerator 105 und dem Gassensor 103 über eine Kommunikationsschnittstelle, wie beispielsweise ein Kabel oder eine Drahtlosverbindung in Verbindung stehen. Die erste Recheneinheit 125 kann einen oder mehrere Prozessoren umfassen, die zum jeweiligen oder gemeinsamen Steuern des Gasgenerators 105 und den Gassensors 103 konfiguriert sind.

Zum Testen des Gasmessgeräts 100 wird durch die ersten Recheneinheit 125 der Gasgenerator 105 derart angesteuert, dass der Gasgenerator 105 in einem ersten Zustand, während eines ersten Zeitabschnitts eines Tests der Testeinheit 101, den Gassensor 103 mittels des ersten Gaskanals 111 mit Testgas beaufschlagt. In einem weiteren Zustand während eines zweiten Zeitabschnitts des Tests steuert die erste Recheneinheit 125 den Gasgenerator 105 derart an, dass dieser den Gassensor 103 mittels des zweiten Gaskanals 113 mit Testgas beaufschlagt.

Da der erste Gaskanal 111 und der zweiten Gaskanal 113 sich in ihrer Kanalstruktur unterscheiden, strömt das Testgas mit unterschiedlicher Transportcharakteristik durch den ersten Gaskanal 111 und den zweiten Gaskanal 113.

Vorliegend tritt das Testgas während des ersten Zeitabschnitts am Ende des ersten Gaskanals 111 in einem geringen Abstand zu dem Einlassbereich 121 des Gassensors 103 aus. Entsprechend strömt das Testgas schnell also direkt zu dem Gassensor 103, wodurch ein Einfluss von Störvariablen, wie beispielsweise Einströmungen eines Umgebungsmediums aus der Umgebung bzw. Ausströmungen von Testgas in die Umgebung, minimal ist. Daher ist eine Messung des Testgases durch den Gassensor 103 in dem ersten Zustand während des ersten Zeitabschnitts einem bezüglich des zweiten Zeitabschnitts reduzierten Einfluss von Störvariablen ausgesetzt. Entsprechend sind durch den Gassensor 103 während des ersten Zustands ermittelte Messwerte besonders valide hinsichtlich einer Funktionalität des Gassensors 103.

In dem weiteren Zustand während des zweiten Zeitabschnitts tritt das Testgas am Ende des zweiten Gaskanals 113 in großem Abstand zum Sensoreinlass 121 und räumlicher Nähe zum Öffnungsbereich 119 aus.

Aufgrund der langen Diffusionswege verbunden mit einer langen Diffusionszeit sind durch den Gassensor 103 während dieses zweiten Zeitabschnitts ermittelte Messwerte besonders anfällig für Störeinflüsse durch Einströmen eines Umgebungsmediums und/oder Ausströmen von Testgas. Insbesondere das Ausströmen des Testgases stellt einen wichtigen Abtransportweg des erzeugten Testgases dar, da sich die Auslassöffnung von 113 in unmittelbarer Nachbarschaft von der dem Öffnungsbereich 119 mit der Gaszutrittsöffnung befindet. Entsprechend sind die während des zweiten Zeitabschnitts ermittelten Messwerte besonders beeinflusst durch und valide hinsichtlich der Detektion einer Verstopfung des Öffnungsbereichs 119.

Entsprechend ist vorgesehen, dass eine Funktionalität des Gassensors 103 anhand von während des ersten Zeitabschnitts ermittelten Messwerten und eine Funktionalität oder eine Durchlässigkeit des Öffnungsbereichs 119 für Gase anhand von während des zweiten Zeitabschnitts ermittelten Messwerten getestet wird.

Zum Analysieren von durch den Gassensor 103 ermittelten Messwerten kann optional neben der ersten Recheneinheit 125 auch eine zweite Recheneinheit 127 vorgesehen werden, die als Analysemodul ausgestaltet ist und mit dem Gassensor 103 über eine Kommunikationsschnittstelle, wie beispielsweise ein Kabel oder eine Drahtlosschnittstelle in kommunikativer Verbindung steht. Selbstverständlich kann zum Analysieren von durch den Gassensor 103 ermittelten Messwerten auch die erste Recheneinheit 125 verwendet werden.

In einem nicht dargestellten Ausführungsbeispiel wird während des ersten Zeitabschnitts ein anderes Testgas durch den entsprechenden Gaskanal, also beispielsweise den ersten Gaskanal geleitet, als während des zweiten Zeitabschnitts. Die beiden verschiedenen Testgase unterscheiden sich vorzugsweise in ihren Diffusionskoeffizienten, so dass bereits durch die verschiedenen Testgase die Transportcharakteristik während des ersten Zeitabschnitts eine andere ist als während des zweiten Zeitabschnitts.

In einem weiteren Ausführungsbeisiel ist lediglich ein einzelner Gaskanal vorgesehen, um die beiden Zeitbereiche mit unterschiedlichen Transportcharakteristika des Testgastransports bereitzustellen. Herbei beruhen die die unterschiedlichen Transportcharakteristika auf unterschiedlichen Testgaseigenschaften, wie beispielsweise auf unterschiedlichen verwendeten testgasen und/oder unterschiedlichen verwendeten Temperaturen von Testgasen.

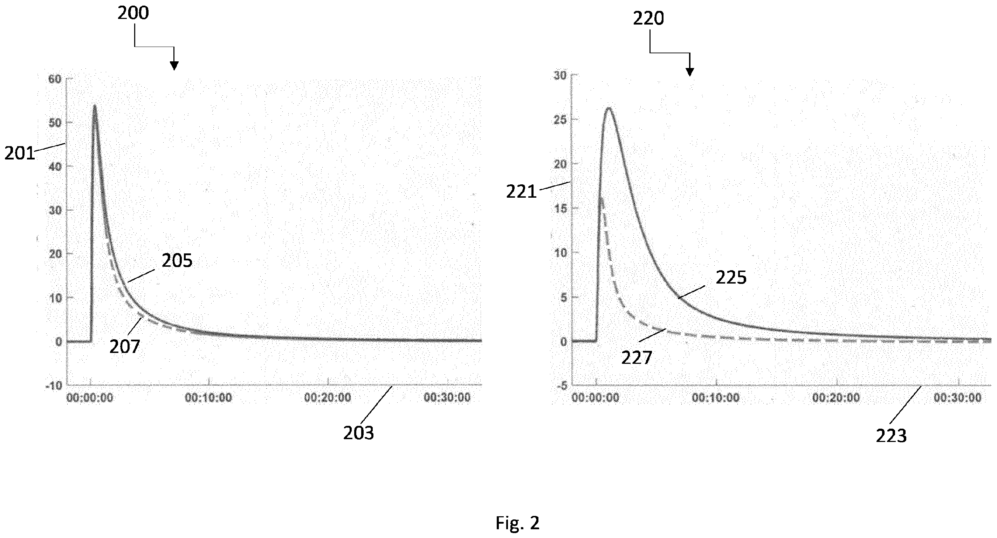

In Fig. 2 ist ein Diagramm 200 dargestellt, das sich auf seiner Ordinate 201 über ein Sensorsignal in [ppm] und auf seiner Abszisse 203 über die Zeit in [hh:mm:ss] aufspannt.

Ein Verlauf 205 stellt Messwerte dar, die von dem Gassensor 103 während einer Begasung mit Testgas in dem ersten Zustand während des ersten Zeitabschnitts aus dem ersten Gaskanal 111 ermittelt wurden und während der Öffnungsbereich 119 für Gase verstopft, also gasundurchlässig, war.

Ein Verlauf 207 stellt Messwerte dar, die von dem Gassensor 103 während einer Begasung mit Testgas in dem ersten Zustand während des ersten Zeitabschnitts aus dem ersten Gaskanal 111 ermittelt wurden und während der Öffnungsbereich 119 durchlässig für Gase, also nicht verstopft, war.

Weiterhin ist in Fig. 2 ein Diagramm 220 dargestellt, das sich auf seiner Ordinate 221 über ein Sensorsignal in [ppm] und auf seiner Abszisse 223 über die Zeit in [hh:mm:ss] aufspannt.

Ein Verlauf 225 stellt Messwerte dar, die von dem Gassensor 203 während einer Begasung mit Testgas in dem weiteren Zustand während des zweiten Zeitabschnitts aus dem zweiten Gaskanal 113 ermittelt wurden und während der Öffnungsbereich 119 für Gase verstopft, also gasundurchlässig, war.

Ein Verlauf 227 stellt Messwerte dar, die von dem Gassensor 103 während einer Begasung mit Testgas in dem weiteren Zustand während des zweiten Zeitabschnitts aus dem zweiten Gaskanal 113 ermittelt wurden und während der Öffnungsbereich 119 durchlässig für Gase, also nicht verstopft, war.

Beim Vergleich der Verläufe 205 und 207 mit den Verläufen 225 und 227 zeigt sich, dass die Verstopfung des Öffnungsbereichs 119 bei einer Begasung mit Testgas durch den zweiten Gaskanal 113 gemäß Verlauf 227 sich deutlich von dem Verlauf 225 unterscheidet. Weiterhin unterscheidet sich die Verstopfung des Öffnungsbereichs 119 bei einer Begasung mit Testgas durch den ersten Gaskanal 111 gemäß Verlauf 205 nur sehr gering von dem Verlauf 207. Entsprechend kann anhand von durch den Gassensor 103 während des weiteren Zustands ermittelten Messwerten gut zwischen einem Zustand, in dem der Öffnungsbereich 119 verstopft ist und einem Zustand, in dem die Öffnung durchlässig für Gase ist, unterschieden werden.

Zum Erkennen eines verstopften Zustands können daher von dem Gassensor 103 während des zweiten Zeitabschnitts ermittelte Messwerte verwendet werden, um eine Abklingzeit, wie beispielsweise eine Halbwertzeit oder einen anderen Wert zu einem vorgegebenen Zeitpunkt nach Start der Begasung zu ermitteln, d.h. zu berechnen oder zu schätzen. Sollte eine Differenz der Abklingzeit zu einem vorgegebenen Referenzwert größer sein als ein vorgegebener Verstopfungsschwellenwert, kann davon ausgegangen werden, dass der Öffnungsbereich 119, insbesondere ein Filter innerhalb des Öffnungsbereichs 119 verstopft ist. Entsprechend ist für diesen Fall vorgesehen, dass die Recheneinheit 125 das Gasmessgerät 100 als verstopft markiert und bspw. eine entsprechende Fehlermeldung in einem Fehlerspeicher ablegt.

Insbesondere kann eine Abklingzeit beispielsweise durch eine Fläche unter einem Maximum zwischen einem vorgegebenen Startzeitpunkt und einem vorgegebenen Stoppzeitpunkt berechnet werden.

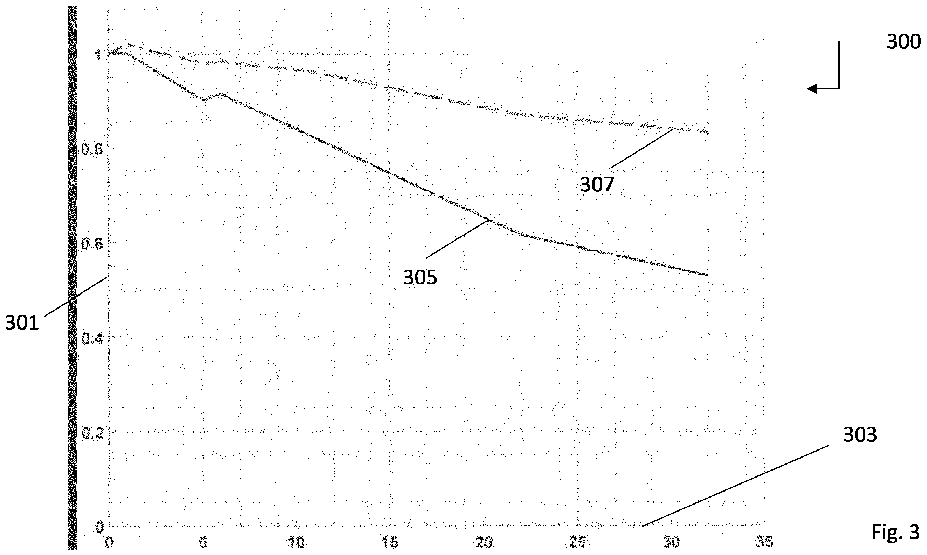

In Fig. 3 ist ein Diagramm 300 dargestellt, das auf seiner Ordinate 301 ein einheitenloses relatives Sensorsignal wiedergibt. Dieses verändert sich durch den Einfluss des Windes für unterschiedliche Windgeschwindigkeiten, die auf der Abszisse 303 in [m/s] abgetragen wurden.

Ein Verlauf 305 basiert auf Messwerten, die von dem Gassensor 103 bei einer Begasung mit Testgas durch den zweiten Gaskanal 113 ermittelt wurden.

Ein Verlauf 307 basiert auf Messwerten, die von dem Gassensor 103 bei einer Begasung mit Testgas durch den ersten Gaskanal 111 ermittelt wurden.

Deutlich erkennbar ist, dass mit zunehmender Windgeschwindigkeit ein Abstand zwischen dem Verlauf 305 und dem Verlauf 307 zunimmt. Dies bedeutet, dass anhand des Abstands zwischen dem Verlauf 305 und dem Verlauf 307 auf eine entsprechende Windgeschwindigkeit geschlossen werden kann und dass die in Verlauf 307 verwendete Anordnung deutlich weniger dem Einfluss von Wind unterliegt und daher geeigneter ist die Sensitivität des Sensors 301 zu überprüfen. Dieser Zusammenhang kann beispielsweise dazu verwendet werden, eine Warnfunktion des Gasmessgeräts 100 zu deaktivieren, wenn Windgeschwindigkeiten ermittelt werden, die größer sind als ein vorgegebener Schwellenwert.

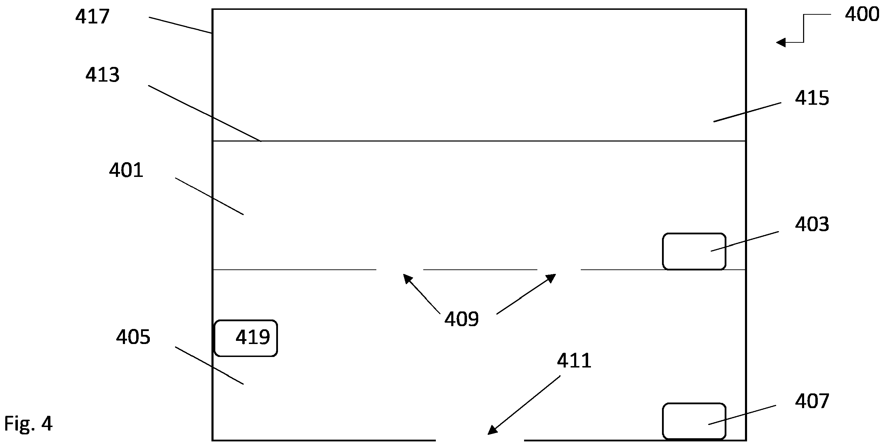

In Fig. 4 ist ein Gasmessgerät 400 dargestellt.

Das Gasmessgerät 400 umfasst einen ersten Gaskanal 401, der hier als Kammer ausgestaltet ist und in dem ein erster Gasgenerator 403 angeordnet ist und eine Recheneinheit 419.

Das Gasmessgerät 400 umfasst weiterhin einen zweiten Gaskanal 405, der hier als Kammer ausgestaltet ist und in dem ein weiterer Gasgenerator 407 angeordnet ist.

Der erste Gaskanal 401 steht mit dem zweiten Gaskanal 405 über Gasübertritte 409 in fluidem Kontakt, sodass ein Gasaustausch zwischen dem ersten Gaskanal 401 und dem zweiten Gaskanal 405 möglich ist.

Der zweiten Gaskanal 405 steht über eine Öffnung 411, die als Sensoreingang dient und in diesem Sinne den Öffnungsbereich für das Gasmessgerät 400 bildet, in fluidem Kontakt mit einer Umgebung, sodass ein direkter, unmittelbarer Gasaustausch zwischen der Umgebung und dem zweiten Gaskanal 405 möglich ist.

Der erste Gaskanal 401 steht mit einer Sensormembran 413 in Kontakt, die einen Elektrolyten eines Gassensors 417 des Gasmessgeräts von dem ersten Gaskanal 401 trennt.

Da der erste Gaskanal 401 nicht direkt mit einer Umgebung in fluidem Kontakt steht, wird ein von dem ersten Gasgenerator 403 bereitgestelltes Testgas nur minimal durch Umgebungsbedingungen des Gasmessgeräts 400, wie beispielsweise Wind, beeinflusst. Entsprechend eignet sich der erste Gaskanal 401 in besonders vorteilhafter Weise zum Testen des Gassensors 417.

Um für den Fall, dass Messwerte des Gassensors 417 bei einer Begasung eines Sensorinnenraums 415 mit Testgas durch den ersten Gasgenerator 403 ergeben, dass der Gassensor 417 als fehlerhaft zu markieren ist, eine falsch negative Fehlermeldung, die den Gassensor 417 fälschlich als fehlerhaft markiert, auszuschließen, kann der erste Gasgenerator 403 mittels des weiteren Gasgenerators 407 getestet werden.

Zum Testen des ersten Gasgenerators 403 ist vorgesehen, dass der Gassensor 417 mit einem durch den weiteren Gasgenerator 407 bereitgestellten Testgas beaufschlagt wird. Für den Fall, dass der Gassensor 417 bei der Begasung mit Testgas durch den weiteren Gasgenerator 407 nicht als fehlerhaft zu markieren ist, ist vorgesehen, dass der Gassensor 417 als fehlerfrei und der erste Gasgenerator 403 als fehlerhaft markiert wird. Dazu kann eine Recheneinheit des Gasmessgeräts 400 eine entsprechende Fehlermeldung in einem Fehlerspeicher des Gasmessgeräts 400 oder in einem Speicher der Recheneinheit ändern bzw. generieren.

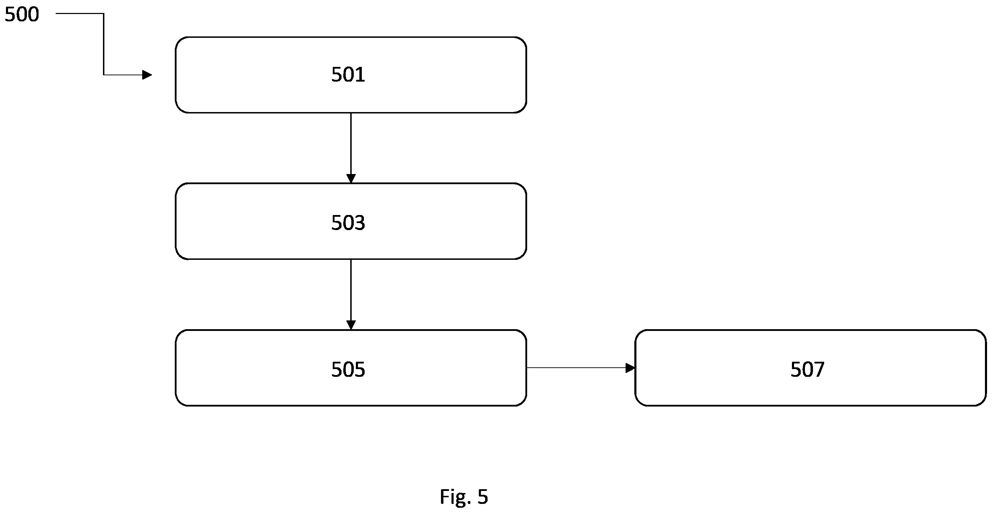

In Fig. 5 ist ein Ablauf des vorgestellten Verfahrens 500 dargestellt.

Das Verfahren 500 startet mit einem Bereitstellungsschritt 501 zum Bereitstellen einer möglichen Ausgestaltung des vorgestellten Gasmessgeräts. Dazu kann beispielsweise eine mögliche Ausgestaltung des vorgestellten Testers mit einem Gassensor zu einer möglichen Ausgestaltung des vorgestellten Gasmessgeräts verbunden werden.

In einem Ansteuerungsschritt 503 wird mindestens ein Gasgenerator des Gasmessgeräts derart angesteuert, dass dieser zu einem ersten Zeitabschnitt eines Tests und zu einem zweiten Zeitabschnitt des Tests über die Gaskanalanordnung den umgebenden Sensorraum des Gassensors mit dem mindestens einen Testgas begast.

In einem Ermittlungsschritt 505, der zumindest zeitweise zeitlich parallel zu dem Ansteuerungsschritt 503 verläuft, werden durch den Gassensor während des ersten Zeitabschnitts und/oder während des zweiten Zeitabschnitts ermittelten Messwerten ermittelt. Dies kann vorteilhaft dadurch geschehen, dass eine Recheneinheit des Gasmessgeräts jeweilige von dem Gassensor ermittelte Messwerte ausliest und in einem Arbeitsspeicher ablegt.

In einem Markierungsschritt 507 wird das Gasmessgerät über eine Testinformation als fehlerhaft markiert, wenn die Auswertealgorithmen eine Abweichung vom Sollzustand ermitteln. Das Markieren erfolgt erfindungsgemäß durch ein Bestimmen und Ausgeben einer Testinformation während des ersten Zeitabschnitts und/oder während des zweiten Zeitabschnitts, wobei die Testinformation indiziert, ob das Gasmessgerät fehlerhaft ist, und wobei das Bestimmen der Testinformation auf einem Vergleich zwischen dem Messwert und einem vorbestimmten Gassensorschwellenwert oder zwischen einem bestimmten Messwertverlaufsparameter und einem vorbestimmten Verlaufsparameter basiert.

### BEZUGSZEICHENLISTE

- 100,400: Gasmessgerät
- 101: Testeinheit
- 103,417: Gassensor
- 105: Gasgenerator
- 107: Basis
- 109: Gaskanalanordnung
- 111, 401: erster Gaskanal
- 113, 405: zweiter Gaskanal
- 115: erste Kammer
- 117: weitere Kammer
- 119: Öffnungsbereich
- 121: Einlassbereich
- 123: Strömungsleitvorrichtung
- 125: erste Recheneinheit
- 127: zweite Recheneinheit
- 129: erste Gasaustrittsöffnung
- 131: weitere Gasaustrittsöffnung
- 200, 220, 300: Diagramm
- 201, 221, 301: Ordinate
- 203, 223, 303: Abszisse
- 205, 207, 225 227, 305, 307: Verlauf
- 403: erster Gasgenerator
- 407: weiterer Gasgenerator
- 409: Gasübertritte
- 411: Öffnung
- 413: Sensormembran
- 415: Sensorinnenraum
- 419: Recheneinheit
- 500: Verfahren
- 501, 503, 504, 507: Verfahrensschritt

## Patentansprüche

1. Tester für ein Gasmessgerät (100), umfassend:
- eine Testeinheit (101), und
- eine Schnittstelle zum reversiblen mechanischen und/oder kommunikativen Verbinden der Testeinheit (101) mit einem Gassensor (103),
wobei die Testeinheit (101) umfasst:
- eine Gaskanalanordnung (109) mit einem ersten Gaskanal (111) und einem zweiten Gaskanal (113), und
- mindestens einen Gasgenerator (105), der dazu eingerichtet ist, mindestens ein Testgas zu einem ersten Zeitabschnitt eines Tests der Testeinheit (101) und zu einem zweiten Zeitabschnitt des Tests über die Gaskanalanordnung (109) in einen umgebenden Sensorraum des Gassensors (103) zu leiten,
wobei die Testeinheit (101) eingerichtet ist, das mindestens eine Testgas zu dem ersten Zeitabschnitt über den ersten Gaskanal (111) zu dem Sensorraum zu leiten und das mindestens eine Testgas zu dem zweiten Zeitabschnitt über den zweiten Gaskanal (113) zu dem Sensorraum zu leiten, und
wobei der erste Gaskanal (111) sich von dem zweiten Gaskanal (113) in seiner Kanalstruktur unterscheidet, so dass ein Testgastransport während des ersten Zeitabschnitts eine andere Transportcharakteristik aufweist als während des zweiten Zeitabschnitts.

2. Tester nach Anspruch 1,
wobei der erste Gaskanal (111) und der zweite Gaskanal (113) sich durch eine Rauheit ihrer Innenoberfläche in ihrer Kanalstruktur voneinander unterscheiden.

3. Tester nach mindestens einem der Ansprüche 1 bis 2,
wobei mindestens ein Gaskanal (111, 113) eine Temperierungseinheit umfasst, die dazu konfiguriert ist, eine Temperatur im Inneren dieses Gaskanals (111, 113) derart zu ändern, dass sich das Testgas zum ersten Zeitabschnitt von dem Testgas zum zweiten Zeitabschnitt in seiner Gastemperatur unterscheidet.

4. Tester nach mindestens einem der Ansprüche 1 bis 3,
wobei der mindestens eine Gasgenerator (105) ausgebildet ist, während des ersten Zeitabschnitts ein anderes Testgas durch den entsprechenden Gaskanal (111, 113) zu leiten als während des zweiten Zeitabschnitts.

5. Tester gemäß Anspruch 4,
wobei ein erstes Testgas während des ersten Zeitabschnitts einen anderen Diffusionskoeffizienten aufweist als ein zweites Testgas während des zweiten Zeitabschnitts.

6. Tester gemäß mindestens einem der vorhergehenden Ansprüche,
wobei der Test durch die Testeinheit (101) weiterhin zu mindestens einem weiteren Zeitabschnitt erfolgt.

7. Gasmessgerät (100), umfassend
- mindestens einen einen Analyten umsetzenden chemischen Gassensor (103) mit einem umgebenden Sensorraum,
- mindestens einen Öffnungsbereich (119), der eine gasdurchlässige Verbindung zwischen dem Sensorraum und einer Umgebung des Gasmessgerätes bildet, und
- mindestens einen Tester nach einem der vorherigen Ansprüche.

8. Gasmessgerät (100) gemäß Anspruch 7,
wobei eine erste Gaskanalöffnung des ersten Gaskanals (111) näher an dem Gassensor (103) liegt als eine zweite Gaskanalöffnung des zweiten Gaskanals (113).

9. Gasmessgerät (100) gemäß Anspruch 7 oder 8,
wobei die zweite Gaskanalöffnung näher an dem mindestens einen Öffnungsbereich (119) liegt als die erste Gaskanalöffnung.

10. Gasmessgerät (100) nach einem der Ansprüche 7 bis 9,
wobei das Gasmessgerät (100) mindestens eine Recheneinheit (125) umfasst, die ausgebildet ist, ein Signal des Gassensors (103) zu empfangen und daraus einen Messwert zu ermitteln, wobei die Recheneinheit (125) weiterhin ausgebildet ist, während des ersten Zeitabschnitts und/oder während des zweiten Zeitabschnitts eine Testinformation zu bestimmen und auszugeben, wobei die Testinformation indiziert, ob das Gasmessgerät (100) fehlerhaft ist, und wobei das Bestimmen der Testinformation auf einem Vergleich zwischen dem Messwert und einem vorbestimmten Gassensorschwellenwert oder zwischen einem bestimmten Messwertverlaufsparameter und einem vorbestimmten Verlaufsparameter basiert.

11. Gasmessgerät (100) gemäß Anspruch 10,
wobei die Testinformation indiziert, ob der mindestens eine Öffnungsbereich (119) verstopft ist und/oder ob der Gassensor (103) funktioniert.

12. Gasmessgerät (100) gemäß mindestens einem der Ansprüche 10 bis 11, wobei der entsprechende durch die Recheneinheit (125) ausgeführte
Vergleich von einer auf dem ermittelten Messwertverlauf basierenden Bestimmung einer Abklingzeit, einer mathematischen Ableitung, eines statistischen Mittels, eines Maximalwerts und/oder eines Integrals abhängig ist.

13. Testverfahren (500) zum Testen eines Gasmessgeräts,
wobei das Testverfahren (500) die folgenden Schritte umfasst:
a) Bereitstellen eines Gasmessgeräts (100) nach einem der Ansprüche 7 bis 12,
b) Ansteuern des mindestens einen Gasgenerators (105) derart, dass dieser mindestens ein Testgas zu einem ersten Zeitabschnitt eines Tests und zu einem zweiten Zeitabschnitt des Tests über die Gaskanalanordnung (109) in den umgebenden Sensorraum des Gassensors (103) leitet,
wobei das mindestens eine Testgas zu dem ersten Zeitabschnitt über den ersten Gaskanal (111) zu dem Sensorraum geleitet wird und zu dem zweiten Zeitabschnitt über den zweiten Gaskanal (113) zu dem Sensorraum geleitet wird,
c) Ermitteln von durch den Gassensor (103) während des ersten Zeitabschnitts und/oder während des zweiten Zeitabschnitts ermittelten Messwerten,
d) Bestimmen und Ausgeben einer Testinformation während des ersten Zeitabschnitts und/oder während des zweiten Zeitabschnitts, wobei die Testinformation indiziert, ob das Gasmessgerät (100) fehlerhaft ist, und wobei das Bestimmen der Testinformation auf einem Vergleich zwischen dem Messwert oder einem daraus berechneten Kennwert und einem vorbestimmten Gassensorschwellenwert oder zwischen einem bestimmten Messwertverlaufsparameter und einem vorbestimmten Verlaufsparameter basiert.

## Claims

1. Tester for a gas measuring device (100), comprising:
- a test unit (101), and
- an interface for reversibly connecting the test unit (101) mechanically and/or communicatively to a gas sensor (103),
wherein the test unit (101) comprises:
- a gas channel arrangement (109) comprising a first gas channel (111) and a second gas channel (113), and
- at least one gas generator (105) that is configured to conduct at least one test gas into a surrounding sensor chamber of the gas sensor (103) via the gas channel arrangement (109) in a first time period of a test of the test unit (101) and in a second time period of the test,
wherein the test unit (101) is configured to conduct the at least one test gas to the sensor chamber via the first gas channel (111) in the first time period and to conduct the at least one test gas to the sensor chamber via the second gas channel (113) in the second time period, and
wherein the first gas channel (111) differs from the second gas channel (113) in its channel structure so that a test gas transport process during the first time period has a different transport characteristic than during the second time period.

2. Tester according to claim 1,
wherein the first gas channel (111) and the second gas channel (113) differ from one another in their channel structure by virtue of a roughness of their inner surface.

3. Tester according to at least one of claims 1 to 2,
wherein at least one gas channel (111, 113) comprises a temperature control unit which is configured to change a temperature in the interior of this gas channel (111, 113) such that the test gas in the first time period differs in its gas temperature from the test gas in the second time period.

4. Tester according to at least one of claims 1 to 3,
wherein the at least one gas generator (105) is designed to conduct a different test gas through the corresponding gas channel (111, 113) during the first time period than during the second time period.

5. Tester according to claim 4,
wherein a first test gas has a different diffusion coefficient during the first time period than a second test gas during the second time period.

6. Tester according to at least one of the preceding claims, wherein the test is further carried out by the test unit (101) in at least one further time period.

7. Gas measuring device (100), comprising
- at least one analyte-converting chemical gas sensor (103) having a surrounding sensor chamber,
- at least one opening region (119) that forms a gas-permeable connection between the sensor chamber and an environment of the gas measuring device, and
- at least one tester according to any of the previous claims.

8. Gas measuring device (100) according to claim 7,
wherein a first gas channel opening of the first gas channel (111) is closer to the gas sensor (103) than a second gas channel opening of the second gas channel (113).

9. Gas measuring device (100) according to claim 7 or 8,
wherein the second gas channel opening is closer to the at least one opening region (119) than the first gas channel opening.

10. Gas measuring device (100) according to any of claims 7 to 9,
wherein the gas measuring device (100) comprises at least one computing unit (125) that is designed to receive a signal from the gas sensor (103) and to determine a measured value from it, wherein the computing unit (125) is further designed to define and output test information during the first time period and/or during the second time period, wherein the test information indicates whether the gas measuring device (100) is faulty, and wherein the definition of the test information is based on a comparison between the measured value and a predefined gas sensor threshold value or between a specific measured value profile parameter and a predefined profile parameter.

11. Gas measuring device (100) according to claim 10,
wherein the test information indicates whether at least one opening region (119) is blocked and/or whether the gas sensor (103) is functioning.

12. Gas measuring device (100) according to at least one of claims 10 to 11, wherein the corresponding comparison performed by the computing unit (125) depends on a definition of a decay time, a mathematical derivation, a statistical average, a maximum value and/or an integral based on the determined measured value profile.

13. Test method (500) for testing a gas measuring device, wherein the test method (500) comprises the following steps:
a) providing a gas measuring device (100) according to any of claim 7 to 12,
b) controlling the at least one gas generator (105) such that it conducts at least one test gas into the surrounding sensor chamber of the gas sensor (103) via the gas channel arrangement (109) in a first time period of a test and in a second time period of the test,
wherein the at least one test gas is conducted to the sensor chamber via the first gas channel (111) in the first time period and to the sensor chamber via the second gas channel (113) in the second time period,
c) determining measured values determined by the gas sensor (103) during the first time period and/or during the second time period,
d) defining and outputting test information during the first time period and/or during the second time period, wherein the test information indicates whether the gas measuring device (100) is faulty, and wherein the definition of the test information is based on a comparison between the measured value or a characteristic value calculated therefrom and a predefined gas sensor threshold value or between a specific measured value profile parameter and a predefined profile parameter.

## Revendications

1. Testeur pour un appareil de mesure de gaz (100), comprenant :
- une unité de test (101), et
- une interface pour la liaison mécanique et/ou de communication réversible de l'unité de test (101) avec un capteur de gaz (103),
dans lequel l'unité de test (101) comprend :
- un agencement de canaux de gaz (109) comportant un premier canal de gaz (111) et un second canal de gaz (113), et
- au moins un générateur de gaz (105) qui est conçu pour conduire au moins un gaz de test, à une première période de temps d'un test de l'unité de test (101) et à une seconde période de temps du test, dans un espace de capteur environnant du capteur de gaz (103) par l'intermédiaire de l'agencement de canaux de gaz (109),
dans lequel l'unité de test (101) est conçue pour conduire l'au moins un gaz de test vers l'espace de capteur par l'intermédiaire du premier canal de gaz (111) lors de la première période de temps et pour conduire l'au moins un gaz de test vers l'espace de capteur par l'intermédiaire du second canal de gaz (113) lors de la seconde période de temps, et
dans lequel le premier canal de gaz (111) diffère du second canal de gaz (113) en ce qui concerne sa structure de canal, de sorte qu'un transport de gaz de test pendant la première période de temps présente une caractéristique de transport différente de celle pendant la seconde période de temps.

2. Testeur selon la revendication 1,
dans lequel le premier canal de gaz (111) et le second canal de gaz (113) se distinguent l'un de l'autre en ce qui concerne leur structure de canal par une rugosité de leur surface intérieure.

3. Testeur selon au moins l'une des revendications 1 à 2,
dans lequel au moins un canal de gaz (111, 113) comprend une unité de régulation de température qui est configurée pour modifier une température à l'intérieur dudit canal de gaz (111, 113) de telle sorte que le gaz de test lors de la première période de temps diffère du gaz de test lors de la seconde période de temps en ce qui concerne sa température de gaz.

4. Testeur selon au moins l'une des revendications 1 à 3,
dans lequel l'au moins un générateur de gaz (105) est conçu pour conduire un gaz de test à travers le canal de gaz (111, 113) correspondant pendant la première période de temps différent de celui conduit pendant la seconde période de temps.

5. Testeur selon la revendication 4,
dans lequel un premier gaz de test pendant la première période de temps présente un coefficient de diffusion différent de celui d'un second gaz de test pendant la seconde période de temps.

6. Testeur selon au moins l'une des revendications précédentes, dans lequel le test est en outre effectué par l'unité de test (101) lors d'au moins une autre période de temps.

7. Appareil de mesure de gaz (100), comprenant
- au moins un capteur de gaz chimique (103) faisant réagir un analyte et comportant un espace de capteur qui l'entoure,
- au moins une zone d'ouverture (119) qui forme une liaison perméable aux gaz entre l'espace de capteur et un environnement de l'appareil de mesure de gaz, et
- au moins un testeur selon l'une des revendications précédentes.

8. Appareil de mesure de gaz (100) selon la revendication 7,
dans lequel une première ouverture de canal de gaz du premier canal de gaz (111) est plus proche du capteur de gaz (103) qu'une seconde ouverture de canal de gaz du second canal de gaz (113).

9. Appareil de mesure de gaz (100) selon la revendication 7 ou 8,
dans lequel la seconde ouverture de canal de gaz est plus proche de l'au moins une zone d'ouverture (119) que la première ouverture de canal de gaz.

10. Appareil de mesure de gaz (100) selon l'une des revendications 7 à 9,
dans lequel l'appareil de mesure de gaz (100) comprend au moins une unité de calcul (125) qui est configurée pour recevoir un signal du capteur de gaz (103) et pour déterminer à partir de celui-ci une valeur de mesure, dans lequel l'unité de calcul (125) est en outre configurée pour déterminer et émettre des informations de test pendant la première période de temps et/ou pendant la seconde période de temps, dans lequel les informations de test indiquent si l'appareil de mesure de gaz (100) est défectueux, et dans lequel la détermination des informations de test est basée sur une comparaison entre la valeur de mesure et une valeur seuil de capteur de gaz prédéterminée ou entre un paramètre d'évolution de valeur de mesure déterminé et un paramètre d'évolution prédéterminé.

11. Appareil de mesure de gaz (100) selon la revendication 10,
dans lequel les informations de test indiquent si l'au moins une zone d'ouverture (119) est obstruée et/ou si le capteur de gaz (103) fonctionne.

12. Appareil de mesure de gaz (100) selon au moins l'une des revendications 10 à 11, dans lequel la comparaison correspondante effectuée par l'unité de calcul (125) dépend d'une détermination d'un temps de décroissance, d'une dérivée mathématique, d'une moyenne statistique, d'une valeur maximale et/ou d'une intégrale basés sur l'évolution de valeur de mesure définie.

13. Procédé de test (500) permettant de tester un appareil de mesure de gaz, dans lequel le procédé de test (500) comprend les étapes suivantes :
a) fourniture d'un appareil de mesure de gaz (100) selon l'une des revendications 7 à 12,
b) commande de l'au moins un générateur de gaz (105) de telle sorte que celui-ci conduit au moins un gaz de test, lors d'une première période de temps d'un test et lors d'une seconde période de temps du test, dans l'espace de capteur environnant du capteur de gaz (103) par l'intermédiaire de l'agencement de canaux de gaz (109),
dans lequel l'au moins un gaz de test est conduit vers l'espace de capteur par l'intermédiaire du premier canal de gaz (111) lors de la première période de temps et est conduit vers l'espace de capteur par l'intermédiaire du second canal de gaz (113) lors de la seconde période de temps,
c) définition de valeurs de mesure définies par le capteur de gaz (103) pendant la première période de temps et/ou pendant la seconde période de temps,
d) détermination et émission d'informations de test pendant la première période de temps et/ou pendant la seconde période de temps, dans lequel les informations de test indiquent si le capteur de gaz (100) est défectueux, et dans lequel la détermination des informations de test est basée sur une comparaison entre la valeur de mesure ou une valeur caractéristique calculée à partir de celle-ci et une valeur seuil de capteur de gaz prédéterminée ou entre un paramètre d'évolution de valeur de mesure déterminé et un paramètre d'évolution prédéterminé.
